# EUROPEAN PATENT APPLICATION

(11) **EP 4 273 791 A1**
(43) Date of publication of application: **08.11.2023**
(21) Application number: 22171722.6
(22) Date of filing: 05.05.2022
(51) Int. Cl.: G06T 5/00, G06T 7/33, G06T 11/00

(54) **METHOD TO RENDER SOFT TISSUE INTO X-RAY MODALITIES**

(71) Applicant: Koninklijke Philips N.V., 5656 AG Eindhoven (NL)
(72) Inventor: GOOSSEN, André, Eindhoven (NL); BYSTROV, Daniel, Eindhoven (NL); FLAESCHNER, Nick, Eindhoven (NL); MAZURKEWITZ, Peter Caesar, Eindhoven (NL); FRERKING, Lena Christina, Eindhoven (NL); KROENKE, Sven, Eindhoven (NL)
(74) Representative: Philips Intellectual Property & Standards

(57) **Abstract**

The present invention relates to a computer-implemented method for rendering soft tissue into an X-ray image. The method comprises the step of providing an articulated three-dimensional anatomical model of at least one joint of a body, wherein the model comprises a rigid structure and at least one type of soft tissue, and wherein the rigid structure comprises at least two bones connected by the joint. The method comprises further the steps of providing an X-ray image of a joint of a patient, wherein the joint of the patient corresponds to the joint of the anatomical model, and registering the anatomical model to the X-ray image. The method comprises further the steps of projecting the at least one type of soft tissue into the image domain of the X-ray image, and visualizing the at least one type of soft tissue in the X-ray image.

## Description

### FIELD OF THE INVENTION

The present invention relates to a computer-implemented method for rendering soft tissue into an X-ray image.

### BACKGROUND OF THE INVENTION

X-ray modalities such as diagnostic X-ray, fluoroscopy, and CT are widely used in the clinical practice and have undergone continuous development in the more than 100 years since the discovery of X-rays. Most recent studies aim at introducing dark field X-ray (DAX) into clinical use in order to visualize diagnostically relevant changes in, for example, lung parenchyma. Contrast agent may help to enhance the radio density in a target tissue or structure. However, many kinds of tissue still remain invisible under X-ray and therefore require additional imaging or surgery to be diagnosed.

Lack of visibility of certain tissues, such as cartilage or tendons makes skeletal X-ray images and sequences incomplete and hampers diagnostic confidence. Often, additional scans in other modalities are required to confirm or contradict a finding. This poses a burden on patients, who have to repeat scanning or see a specialized radiography department, and staff, who have to repeat scans for the same patient. In addition, also care providers are burdened, who may experience a higher machine load. Ideally, some medical conditions could directly be assessed from routine X-ray images that cannot be diagnosed up to now.

The inventors of the present invention have thus found that it would be advantageous to have a method for rendering soft tissue into an X-ray image that does not suffer from the above-mentioned drawbacks.

### SUMMARY OF THE INVENTION

It is an object of the present invention to provide a method for rendering soft tissue into an X-ray image.

The object of the present invention is solved by the subject matter of the independent claims, wherein further embodiments are incorporated in the dependent claims.

The described embodiments similarly pertain to the computer-implemented method for rendering soft tissue into an X-ray image, the data processing apparatus, the computer program and the computer-readable storage medium. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method embodiment, unless explicitly mentioned to the contrary hereinafter.

According to a first embodiment of the invention, there is provided a computer-implemented method for rendering soft tissue into an X-ray image. The method comprises the step of providing an articulated three-dimensional anatomical model of at least one joint of a body, wherein the model comprises a rigid structure and at least one type of soft tissue, and wherein the rigid structure comprises at least two bones connected by the joint. The method comprises further the steps of providing an X-ray image of a joint of a patient, wherein the joint of the patient corresponds to the joint of the anatomical model, and registering the anatomical model to the X-ray image. The method comprises further the steps of projecting the at least one type of soft tissue into the image domain of the X-ray image, and visualizing the at least one type of soft tissue in the X-ray image.

Thus, according to this method, an articulated anatomical model comprising at least one rigid and one non-rigid set of structures is provided, as well as a medical X-ray image. The model and the X-ray image cover preferably the same region of a body of a patient, in particular the same joint like the knee joint, for example. The anatomical model comprises at least a rigid structure and at least one type of soft tissue, wherein the rigid structure can comprise a plurality of bones, which are connected with each other by a joint. The soft tissue can be regarded as the part of the body of the patient that are not bones, or at least a part of that. Soft tissue can be, for example, tendons, ligaments, skin, cartilage, muscles, etc. In particular, according to the invention, soft tissue can be regarded as tissue with a relatively low X-ray absorption coefficient, such that soft tissue is not clearly imaged in X-ray images. In addition, a lack of difference in attenuation of different soft tissue types like muscle, fat, or cartilage contributes to making it difficult to image soft tissue in skeletal radiography. The articulated three-dimensional anatomical model can be a computer-implemented representation of at least one joint of a body, wherein the model comprises the relative position and orientation of the bones of the rigid structure and the soft tissue with respect to each other over an articulation range of the joint. The articulation range and thus the posture of rigid as well as soft parts of the model can be sampled as sampling/supporting points over the range of articulation of the anatomy of interest, for example straight leg to full flexion for the knee joint.

In the step of registering the anatomical model to the X-ray image, the anatomical model can be adapted to the posture of the joint depicted in the X-ray image, and the correct position and orientation of the model can determined such that the rigid structure of the anatomical model corresponds with the bones of the X-ray image. In the next step, the at least one type of soft tissue is derived from the anatomical model, and the soft tissue is forward projected into the image domain of the X-ray image. Thus, the soft tissue can be rendered and/or visualized in the X-ray image together with the bones of the X-ray image. This step can be repeated for a plurality of images in a time series of images in the study under examination.

The articulated anatomical 3D model offers a good understanding on how those soft tissue parts look and behave in different postures and offers the possibility to model those soft tissue components using the model comprising both, rigid and non-rigid structures. The registration based on rigid structures that are visible in X-ray examinations, and rendering non-rigid structures, which are mostly non-visible in X-ray exams, can thus form a powerful enhancement of classical X-ray images in diagnostics, fluoroscopy, tomosynthesis or a (cone-beam) CT. These images that are enhanced according to the invention boost the readability - especially for less trained readers - and can thus improve diagnostic confidence. By mapping a statistical atlas for a certain structure of interest into the X-ray image and local adaptation of the tone or color map any deviations and abnormalities can be visually highlighted.

This method can be applied, for example, as a software feature in quality control, CAD, PACS, in a viewing station or as an educational product. However, the method can be applied to any X-ray imaging modality in two or three dimensions in static or dynamic imaging.

In an embodiment of the invention, the step of registering the anatomical model to the X-ray image comprises the steps of segmenting the X-ray image to determine a position of at least two bones in the X-ray image, aligning the rigid structure of the anatomical model with the position of the at least two bones in the X-ray image by applying a transformation to the anatomical model, wherein the transformation is defined by rotation, scaling, and translation of the anatomical model and an adjustment of the posture of the joint of the anatomical model, and determining a position of the at least one type of soft tissue of the anatomical model after the transformation is applied to the anatomical model.

Thus, an articulated 3D model of the target anatomy can be statically or dynamically registered to an X-ray image. Segmentation of the bones in the X-ray image can be performed, for example, via a model-based segmentation, a fully convolutional neural network, or other well-known approaches. Alignment of the rigid parts of the articulated model with the segmented bones can be performed by optimizing rotation, scale, translation and preferably an articulation and posture of the respective joint simultaneously. For two-dimensional images this may require a pose estimation of the joint in the image.

Selected soft tissue body parts can then be taken from the respectively articulated model and transformed using the same transformation in order to render them directly into the X-ray image domain or map a statistical probability map of presence of the structure of interest from a statistical anatomical atlas into the X-ray in order to enhance the visibility of these structures using a locally adapted color or tone map. Soft tissue components can be added by deforming interpolated surfaces or volumes of soft tissue by the transform determined before. The soft tissue can then be forward projected into the image domain either using the CT or X-ray geometry and/or the pose deduced before. Different ways of visualization these additional soft tissue parts can be utilized.

Furthermore, if an image is acquired with a different modality, structures like soft tissue can be mapped to the X-ray image, by adapting the articulated model to this second image and compensating the possible different articulations of both images.

In an embodiment of the invention, the step of providing an articulated three-dimensional anatomical model comprises providing a plurality of articulated three-dimensional anatomical models, and the step of aligning the rigid structure of the anatomical model with the position of the at least two bones in the X-ray image comprises the steps of determining a residual error for each of the anatomical model of the plurality of anatomical models, and selecting the anatomical model of the plurality of anatomical models with the smallest residual error.

Thus, it is possible to provide a suitable model for each patient. Providing a plurality of anatomical models ensures that a model can be determined and used that corresponds best to the anatomy of the respective patient.

In an embodiment of the invention, the plurality of anatomical models comprises a male model and a female model.

In addition to male and female anatomical models, also models for different age of the patient can be provided, for example a plurality of anatomical models of children or teenagers. In addition, it is possible to provide respective models for patients with different body mass index like underweight, normal, obese, or athletic patients. Using a plurality of models ensures best results of the visualization of the soft tissue in the X-ray image.

In an embodiment of the invention, the X-ray image is a two-dimensional image, and the step of aligning the rigid structure of the anatomical model with the position of the at least two bones in the X-ray image comprises estimating a pose of the joint of the patient in the X-ray image.

Using a two-dimensional X-ray image might require first determining a pose of the joint in the X-ray image before the rigid structure ca be aligned with the bones of the X-ray image.

In an embodiment of the invention, the X-ray image is a three-dimensional X-ray image. Thus, also images of, for example, computed tomography examinations can be used and enhanced with the method according to the invention.

In an embodiment of the invention, the joint is a hip joint, a knee joint, an ankle joint, a shoulder joint, an elbow joint, or a wrist joint, and/or the soft tissue comprises a ligament, a muscle, a cartilage, a tendon, or a skin.

According to the invention, the articulated three-dimensional anatomical model can be the model of a human joint. The joint can be but is not limited to hip joint, knee joint, ankle joint, shoulder joint, elbow joint, wrist joint. The model at least comprises the main bones as rigid parts, potentially additional bones, such as the patella for the knee joint, and at least one type of soft tissue, e.g. but not limited to ligaments, muscles, cartilage, skin.

In an embodiment of the invention, the articulated three-dimensional anatomical model is built from a dedicated or routine examination of a study subject and/or from an anatomical atlas.

In an embodiment of the invention, the articulated three-dimensional anatomical model comprises a position and a shape of the rigid structure and the at least one type of soft tissue over an articulation range of the joint.

In an embodiment of the invention, the step of segmenting the X-ray image is performed by a model-based segmentation or by a fully convolutional neural network.

Alternatively, the step of registering the anatomical model to the X-ray image can be performed via an end-to-end deep-learned model. Thus, the parameters of the registration like rotation, scaling, translation and posture can be derived from the deep-learned model. In an embodiment of the invention, the step of visualizing the at least one type of soft tissue in the X-ray image comprises determining an intensity change in the X-ray image and adapting the visualization of the at least one type of soft tissue based on the determined intensity change.
In this embodiment, soft tissue parts are post-processed using low level intensity changes in the original image and thus refined to the actual image content.

Further, in an embodiment of the invention, the enhancing of X-ray images by soft-tissue structures deduced from an articulated 3D model of the anatomy can be used for automatic region-of-interest (ROI) localization. These ROIs can be utilized to draw the attention of the reader to certain regions in the image or as a preprocessing step for computer-assisted-diagnosis applications.

In an embodiment of the invention, the step of visualizing the at least one type of soft tissue in the X-ray image comprises rendering the soft tissue as an additional contrast, tone mapping, color overlay, or hue modulation.

Thus, soft tissue body parts can be visualized as an overlay, in color or intensity modulation or a side-by-side view. Soft tissue parts can also be rendered using tone mapping to enhance the dynamic range of the image. For image sequences soft tissue dynamics can be rendered into each frame.

According to another aspect of the invention, there is provided a data processing apparatus comprising a processor configured to perform the steps of the method of any of the preceding embodiments.

The data processing apparatus can comprise a plurality of processors to execute the computer program according to the invention.

According to another aspect of the invention, there is provided a computer program comprising instructions which, when the program is executed by a computer, cause the computer to carry out the steps of the method of any of the preceding embodiments.

The computer program can be performed on one or more processing units, which are instructed to cause the method for rendering soft tissue into an X-ray image. Preferably, the program is stored in a system for rendering soft tissue into an X-ray image and a processing unit carrying out this program is part of said system. The computer program may be part of a computer program, but it can also be an entire program by itself. For example, the computer program may be used to update an already existing computer program to get to the present invention.

According to another aspect of the invention, there is provided a computer-readable storage medium comprising instructions which, when executed by a computer, cause the computer to carry out the steps of the method of any of the preceding embodiments.

The computer program may be stored on a computer-readable storage medium. The computer-readable storage medium may be seen as a storage medium, such as for example, a USB stick, a CD, a DVD, a data storage device, a hard disk, or any other medium on which a program as described above can be stored.

Thus, the benefits provided by any of the above aspects equally apply to all of the other aspects and vice versa.

In a gist, the invention relates to a computer-implemented method for rendering soft tissue into an X-ray image. The method comprises the step of providing an articulated three-dimensional anatomical model of at least one joint of a body, wherein the model comprises a rigid structure and at least one type of soft tissue, and wherein the rigid structure comprises at least two bones connected by the joint. The method comprises further the steps of providing an X-ray image of a joint of a patient, wherein the joint of the patient corresponds to the joint of the anatomical model, and registering the anatomical model to the X-ray image. The method comprises further the steps of projecting the at least one type of soft tissue into the image domain of the X-ray image, and visualizing the at least one type of soft tissue in the X-ray image.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows an articulated three-dimensional anatomical model of the rigid structure of a knee.
Fig. 2A shows an articulated three-dimensional anatomical model of a type of soft tissue of an ankle.
Fig. 2B shows an articulated three-dimensional anatomical model of the rigid structure of an ankle.
Fig. 3A shows an X-ray image of a knee joint.
Fig. 3B shows the rigid structure of an articulated three-dimensional anatomical model registered to the bones of an X-ray image of the knee joint.
Fig. 4 shows on the left side a lateral X-ray image of a knee, and on the right side the X-ray image of the knee with enhanced visualization of soft tissue like patellar and quadriceps femoris tendons and Hoffa's fat pad.
Fig. 5 shows a block diagram of a method for rendering soft tissue into an X-ray image according to an embodiment of the invention.

### DETAILED DESCRIPTION OF EMBODIMENTS

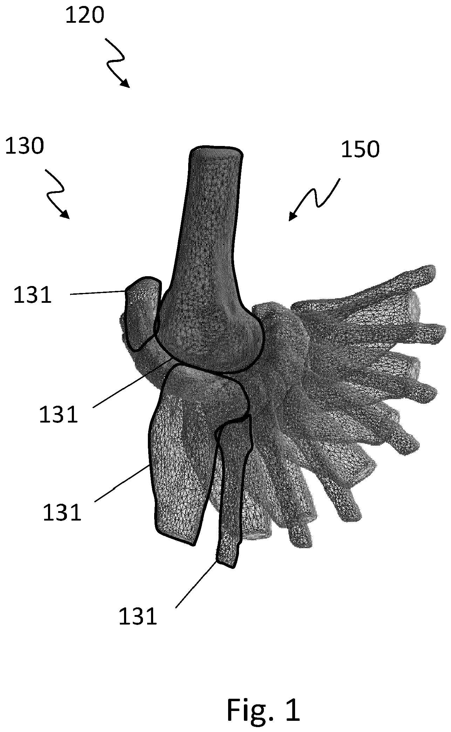

Fig. 1 shows an example of an articulated three-dimensional anatomical model (120) of the rigid structure (130) of a knee joint (150) over an articulation range of the knee. In the straight position, the bones (131) are emphasized with the black edging in order to improve visibility of femur, tibia, fibula and patella. Further, in this figure, the position of the rigid structure (130) of tibia, fibula and patella are indicated over various flexion angles up to full flexion of the knee joint. These bones can be used for registration into X-ray image domains.

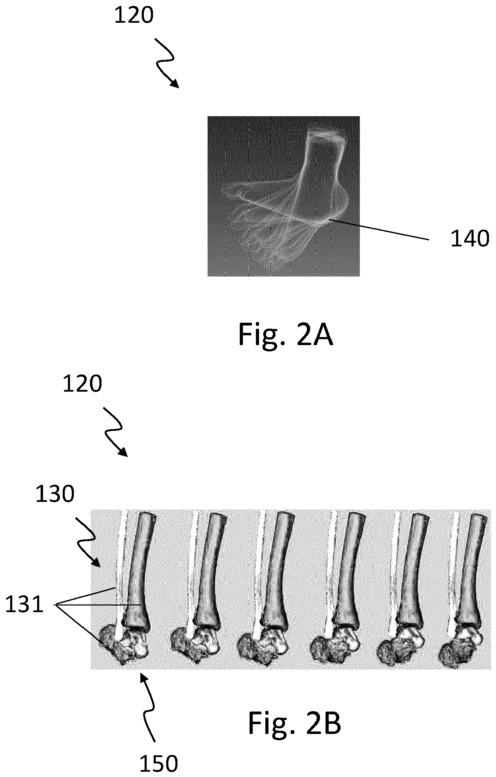

Fig. 2A shows an articulated three-dimensional anatomical model (120) of a type of soft tissue (140) of an ankle joint (150). This is an example for an articulation of non-rigid parts of the ankle, the skin surface as a type of soft tissue is visualized for different flexion angles of the ankle joint.
Fig. 2B shows an articulated three-dimensional anatomical model (120) of the rigid structure (130) of an ankle joint (150). Different bones (131) like tibia, fibula and parts of the foot are visualized over an articulation range of the ankle. Knowing the form of the skin surface, for example, in dependence of the posture of rigid parts allows for morphing the skin surface to the estimated bone constellation in a given X-ray image. A kinematic model of further soft-tissue structures such as ligaments or muscles being coupled to the bone and skin constellation allows for predicting also the spatial constellation of these additional structures.

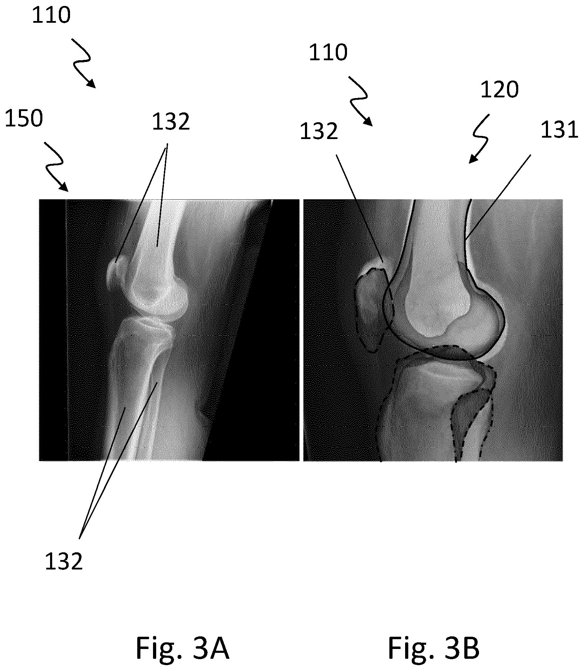

Fig. 3A shows an X-ray image (110) of a knee joint (150). The bones (132) are clearly visible in the X-ray image due to their higher specific X-ray absorption coefficient, whereas soft tissue structures are not clearly visible in such an X-ray image.

Fig. 3B shows the rigid structure (130) with the bones (131) of an articulated three-dimensional anatomical model (120) registered to the bones (132) of the X-ray image (110) of the knee joint (150) of Fig. 3A. The white regions indicate the bones (132) detected by the X-ray image, whereas the black edging indicates the surface of the bones (131) of the rigid structure (130) of the articulated three-dimensional anatomical model (120) of the knee joint (150) that is registered to the X-ray image (110).

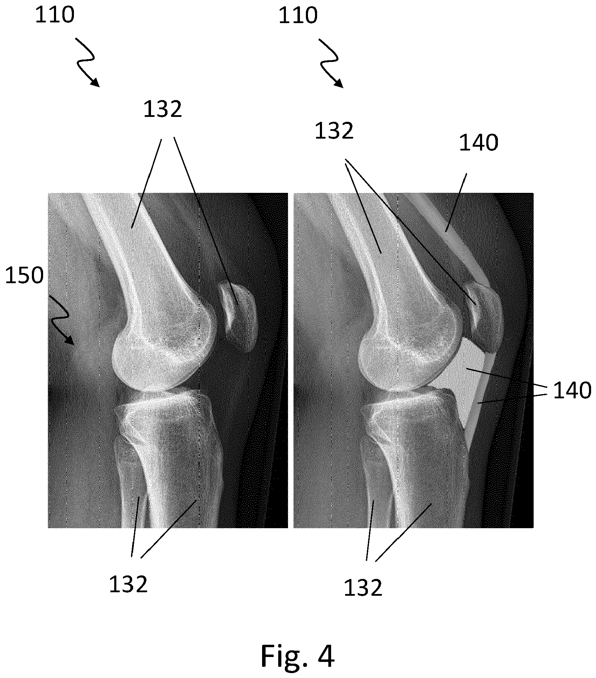

Fig. 4 shows on the left side a lateral X-ray image (110) of a knee joint (150) with the bones (132) of femur, tibia, fibula, and patella being clearly visible, whereas soft tissue is only slightly visible. On the right hand side, Fig. 4 shows the same X-ray image as on the left hand side. However, on the right hand side, the X-ray image is enhanced according to the method of the present invention. The soft tissue structures (140) of the knee joint like patellar and quadriceps femoris tendons and Hoffa's fat pad are taken from the registered anatomical model (120) and projected into the X-ray image domain. These soft tissue structures (140) are then visualized in the X-ray image as an additional overlay. In this example, especially the patellar tendon is enhanced visualized, which connects the patella to the tibia.

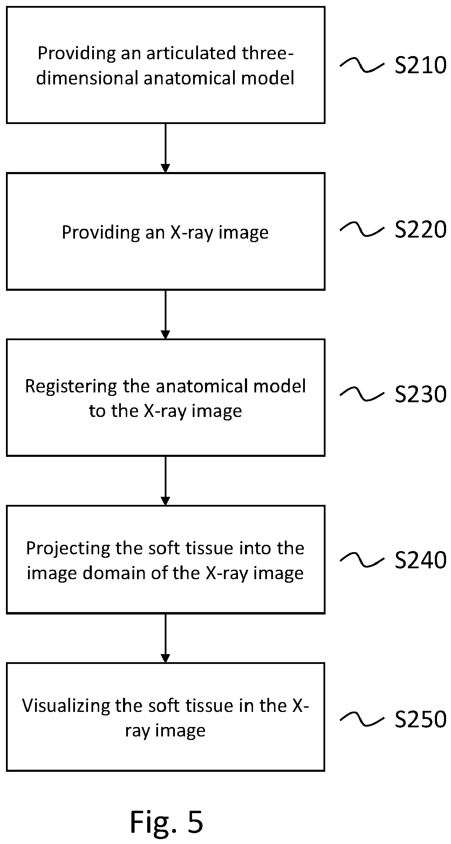

Fig. 5 shows a block diagram of a method for rendering soft tissue (140) into an X-ray image (110) according to an embodiment of the invention. After an articulated three-dimensional anatomical model (120) of at least one joint (150) of a body is provided (S210) and an X-ray image (110) of the respective joint (150) is provided (S220), the anatomical model is registered to the X-ray image (S230). In the next step, the soft tissue (140) is projected into the image domain of the X-ray image (S240), and the soft tissue is visualized in the X-ray image (S250).

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 110: X-ray image
- 120: articulated three-dimensional anatomical model
- 130: rigid structure
- 131: bones of the anatomical model
- 132: bones in the X-ray image
- 140: soft tissue
- 150: joint

## Claims

1. A computer-implemented method for rendering soft tissue (140) into an X-ray image (110), the method comprising the steps of:
providing (S210) an articulated three-dimensional anatomical model (120) of at least one joint (150) of a body, wherein the model comprises a rigid structure (130) and at least one type of soft tissue (140), and wherein the rigid structure (130) comprises at least two bones (131) connected by the joint (150);
providing (S220) an X-ray image (110) of a joint (150) of a patient, wherein the joint (150) of the patient corresponds to the joint (150) of the anatomical model (120);
registering (S230) the anatomical model (120) to the X-ray image (110);
projecting (S240) the at least one type of soft tissue (140) into the image domain of the X-ray image (110); and
visualizing (S250) the at least one type of soft tissue (140) in the X-ray image (110).

2. The method according to claim 1, wherein the step of registering (S230) the anatomical model (120) to the X-ray image (110) comprises the steps of
segmenting the X-ray image (110) to determine a position of at least two bones (132) in the X-ray image;
aligning the rigid structure (130) of the anatomical model (120) with the position of the at least two bones (132) in the X-ray image (110) by applying a transformation to the anatomical model (120), wherein the transformation is defined by rotation, scaling, and translation of the anatomical model and an adjustment of the posture of the joint (150) of the anatomical model (120); and
determining a position of the at least one type of soft tissue (140) of the anatomical model (120) after the transformation is applied to the anatomical model.

3. The method according to claim 2,
wherein the step of providing (S110) an articulated three-dimensional anatomical model (120) comprises providing a plurality of articulated three-dimensional anatomical models, and
wherein the step of aligning the rigid structure (130) of the anatomical model with the position of the at least two bones (132) in the X-ray image comprises the steps of
determining a residual error for each of the anatomical model of the plurality of anatomical models, and
selecting the anatomical model of the plurality of anatomical models with the smallest residual error.

4. The method according to claim 3, wherein the plurality of anatomical models comprises a male model and a female model.

5. The method according to any of claims 2 to 4, wherein the X-ray image (110) is a two-dimensional image, and wherein the step of aligning the rigid structure (130) of the anatomical model (120) with the position of the at least two bones (132) in the X-ray image (110) comprises estimating a pose of the joint (150) of the patient in the X-ray image (110).

6. The method according to any of claims 1 to 4, wherein the X-ray image (110) is a three-dimensional X-ray image.

7. The method according to any of the preceding claims, wherein the joint (150) is a hip joint, a knee joint, an ankle joint, a shoulder joint, an elbow joint, or a wrist joint, and/or wherein the soft tissue (140) comprises a ligament, a muscle, a cartilage, a tendon, or a skin.

8. The method according to any of the preceding claims, wherein the articulated three-dimensional anatomical model (120) is built from a dedicated or routine examination of a study subject and/or from an anatomical atlas.

9. The method according to any of the preceding claims, wherein the articulated three-dimensional anatomical model (120) comprises a position and a shape of the rigid structure (130) and the at least one type of soft tissue (140) over an articulation range of the joint (150).

10. The method according to any of the preceding claims, wherein the step of segmenting the X-ray image (110) is performed by a model-based segmentation or by a fully convolutional neural network.

11. The method according to any of the preceding claims, wherein the step of visualizing (S250) the at least one type of soft tissue (140) in the X-ray image (110) comprises determining an intensity change in the X-ray image (110) and adapting the visualization of the at least one type of soft tissue (140) based on the determined intensity change.

12. The method according to any of the preceding claims, wherein the step of visualizing (S250) the at least one type of soft tissue (140) in the X-ray image (110) comprises rendering the soft tissue as an additional contrast, tone mapping, color overlay, or hue modulation.

13. A data processing apparatus comprising a processor configured to perform the steps of the method of any of claims 1 to 12.

14. A computer program comprising instructions which, when the program is executed by a computer, cause the computer to carry out the steps of the method of any of claims 1 to 12.

15. A computer-readable storage medium comprising instructions which, when executed by a computer, cause the computer to carry out the steps of the method of any of claims 1 to 12.
